# EUROPEAN PATENT SPECIFICATION

(11) **EP 2 723 249 B1**
(45) Date of publication and mention of the grant of the patent: **20.12.2017**
(21) Application number: 12728897.5
(22) Date of filing: 07.06.2012
(51) Int. Cl.: A61B 17/072

(54) **CARTRIDGE ASSEMBLY**
PATRONENANORDNUNG
ENSEMBLE CARTOUCHE

(30) Priority: 21.06.2011 CN 201120220904 U
(43) Date of publication of application: 30.04.2014
(73) Proprietor: Ethicon Endo-Surgery, Inc., Cincinnati, OH 45242 (US)
(72) Inventor: CHEN, Siliang, Pudong Shanghai 200030 (CN); VASUDEVAN, Venkataramanan, Mandakolathur, Mumbai 400 016 (IN)
(74) Representative: Tunstall, Christopher Stephen
(86) International application number: PCT/US2012/041312
(87) International publication number: WO 2012/177408

(56) References cited:
- WO-A1-03/094746
- WO-A2-03/079909
- WO-A2-2006/049852
- US-A- 5 413 267

## Description

### FIELD

The present disclosure generally relates to a linear surgical stapling instrument, and more particularly, to improvements to a cartridge assembly for a linear surgical stapling instrument.

### BACKGROUND

More and more surgeons tend to use surgical staples, rather than conventional sutures since the use of surgical staples can make many complex procedures much simpler and thereby reduce operation time. Many types of surgical staplers have been devised for different procedures, including linear staplers.

These instruments generally include a pair of jaws cooperating with each other and supporting anvil and staple cartridge, respectively. The instruments also include a driver within a cartridge casing, wherein the driver pushes all of the staples out simultaneously against the anvil to form the staples into a generally B-shape, suturing tissue together. In addition, these instruments include an approximation mechanism that allows for a relative movement between the cartridge and anvil to receive tissue therebetween. Finally, the instruments include a handle and a firing mechanism. A surgeon moves the driver forward to form the staples against the anvil by actuating the firing mechanism with a firing trigger.

A significant improvement to linear surgical stapling instruments is the provision of a pre-sterilized, disposable loading unit or a cartridge assembly. This substantially reduces the using cost of the linear surgical stapling instrument and increases its service life. It is easy to understand the forming ability of the surgical staples has a direct influence on the tissue stapling effect. It has been found that those surgical staples well lubricated can form better when being fired.

The present disclosure is directed to provide a cartridge assembly in which the surgical staples loaded may be better lubricated to form more effectively.

WO2006049852 describes surgical apparatus including a disposable loading unit selectively operatively engagable within a distal end of a first half-section; and a wound treatment material applicator assembly for delivering wound treatment material to the target surgical site. The disposable loading unit includes a cartridge; a plurality of deployable needles supported within the cartridge, wherein each needle includes a lumen extending therethrough and at least one hole formed in an outer periphery thereof; and an actuation member translatably disposed within the cartridge for delivering a driving force to each needle to deploy the needles from the cartridge. The applicator assembly includes a first and second reservoir supported on the distal end of a respective first and second half-section; and a source of wound treatment material in fluid communication with each reservoir.

US5413267 describes a stapler that includes a mechanism for sensing whether the stapler is loaded with a fired cartridge housing and for preventing the stapler from being closed or fired when loaded with the fired cartridge.

### SUMMARY

The present invention is defined by the independent claim with further details of exemplary embodiments being described by the dependent claims.

### BRIEF DESCRIPTION OF THE DRAWINGS

Other features and advantages of the present disclosure will become apparent from the following detailed description when viewed in conjunction with the accompanying drawings, which set forth certain exemplary but not-limiting embodiments of the disclosure;
Fig. 1 is a perspective view of the cartridge assembly according to one embodiment of the present disclosre;
Fig. 2 is an exploded view of the cartridge assembly;

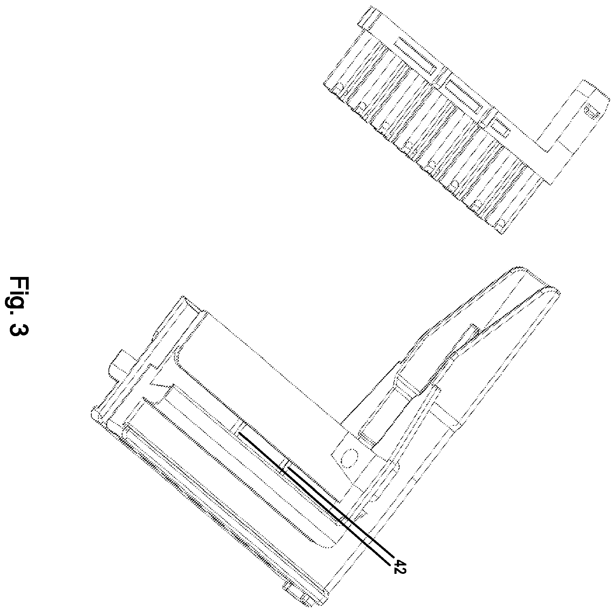

Fig.3 is another exploded view of the cartridge assembly, showing the openings on a casing;
Fig.4 is a schematic perspective view of a linear stapler including a cartridge assembly according to the present disclosure; and

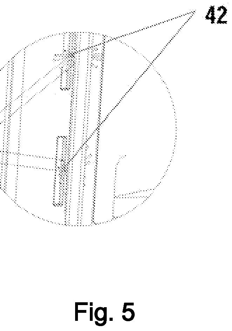

Fig.5 is an enlarged view of the section A in Fig.4.

### DESCRIPTION OF THE PREFERRED EMBODIMENTS

In the following, certain exemplary embodiments of the present disclosure will be described in detail with reference to the accompanying drawings, in which like numeral signs are used for like components.

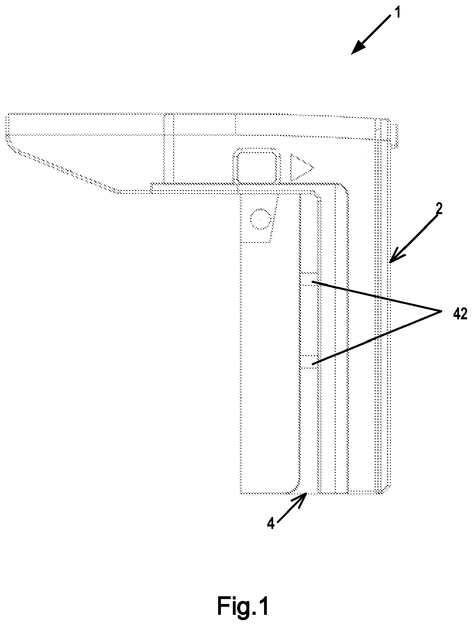

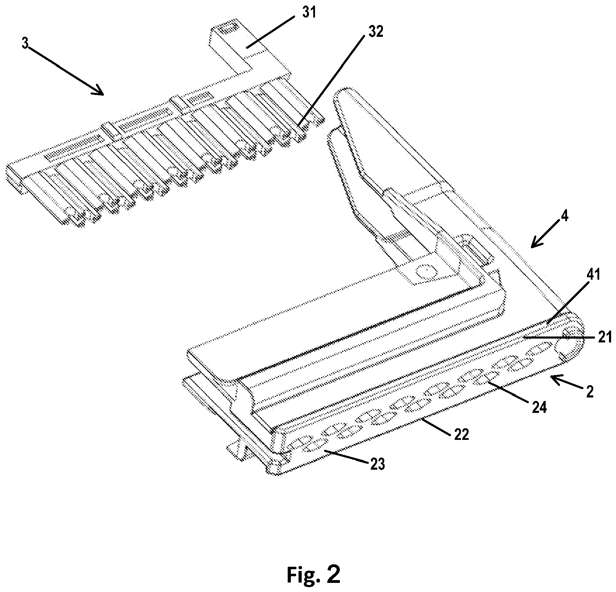

Referring to Fig.1, a cartridge assembly 1 adapted to achieve the unique advantages of the present utility model is illustrated. The cartridge assembly 1 includes a cartridge 2, a staple driver 3 (not shown in Fig.1, see Fig.2) and a casing 4. Referring to Fig.2, the cartridge 2 includes an elongate body 22 having an upstanding surrounding flange 21. When in use, a forward surface 23 of the cartridge 2 constitutes a surface which comes into contact with tissue. The cartridge 2 is provided with a plurality of staple-containing slots 24 extending longitudinally through the forward surface 23. Each staple-containing slot 24 is used to frictionally receive a surgical staple, for example.

Referring to Fig.2, in one embodiment, the staple driver 3 includes an elongate plate-like driver body 31 and a plurality of staggered tips 32 extending forwardly from the driver body 31. The tips 32 are equal in number to the staples loaded in the cartridge 2. The tips 32 are insertable into the corresponding staple-containing slots 24 of the cartridge 2 to align with the surgical staples. As the staple driver 3 moves forwardly, these tips 32 push all the surgical staples contained in the staple-containing slots 24 toward an anvil (not shown) of a linear stapling instrument, thereby forming the surgical staples.

The casing 4 is an elongate hollow housing defining a chamber therein. The forward end of the casing 4 is open and is surrounded by a flange 41. The flange 41 may fit within the flange 21 of the cartridge 2, such that the cartridge 2 and the casing 4 may be joined together through wielding, gluing or the like. The staple driver 3 is housed in the casing 4 and located behind the cartridge 2.

As described above, for various factors, the cartridge assembly 1 constituted by the cartridge 2, the staple driver 3 and the casing 4 is usually formed as a disposable assembly which is disposed of after a single use. To facilitate the formation of the staple when being fired, it is common to immerse the duly assembled cartridge assembly 1 into a lubricant to perform staple lubrication process during manufacture of the disposable cartridge assembly 1. The cartridge assembly 1 is generally dimensioned under strict limitation in consideration of the invasion of the procedures, which requires the driver body 31 to contact closely with a wall of the casing 4. As a result, the lubricant may only lubricate the loaded surgical staples through the staple-containing slots 24 in the front of the cartridge 1 during the lubricating process. Therefore, these staples can't be lubricated well even if the cartridge assembly is immersed in the lubricant for a long time. That is to say, the efficiency and quality of the manufacture are both frustrating. For the same reasons, it is not easy for the cartridge assembly after being lubricated to get dry, and it takes quite a long time for this kind of cartridge assembly to drain the lubricant.

In view of the above, as shown, the present disclosure provides one or more (for example, two) openings 42 in an area of the side of the casing 4 corresponding to those tips 32 of the staple driver 3. By doing this, the lubricant may flow into the cartridge assembly via these openings 42 without being hindered by the driver body 31, and then flows through a gap between the tips 32 to the surgical staples in the staple-containing slots 24 to lubricate the surgical staples. As such, when the cartridge assembly is immersed into the lubricant, the lubricant may lubricate the surgical staples effectively both in forward and rearward directions through the staple-containing slots 24 and openings 42 in a short time. Also, the lubricant may leave the cartridge assembly simultaneously via the staple-containing slots 24 and openings 42 when needed, thus drying the cartridge assembly more quickly. Of course, the provision of two openings is only exemplary, one and more openings can also be provided. Moreover, the casing 4 may be provided with one or more such openings at corresponding positions on the other side of the casing 4.

The openings 42 may be embodied as various forms such as chambers, cavities, gaps, hollow spaces, holes, orifices, slots, recesses, outlets, etc to facilitate the staple lubrication. Besides, the openings may have any suitable shape, such as rectangular, circular and annular shape, etc. With the casing 4 having openings 42 on its associated part, various coating processes may be used to spray lubricant during the formation of the cartridge. For example, a roll coating process may be employed so that through such openings, the lubricant may get sprayed onto the staples loaded in the staple cartridge. The lubricant can be a solid lubricant such as molybdenum or its composite. For example, in one embodiment, molybdenum sulfide may be used as the lubricant. The lubricant can also be an oil-based lubricant such as lubricating oil commonly used in the industry. It can be appreciated that any other organic or inorganic viscous fluids can be used as the lubricant so long as they can provide a lubrication effect to facilitate the staple firing.

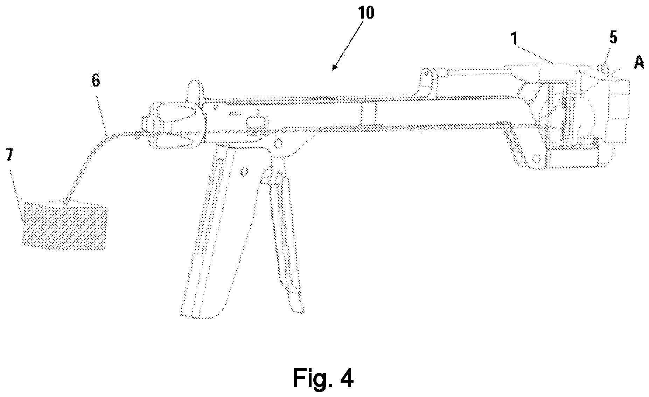

In addition to being used as lubricating holes, the openings 42 on the casing 4 may also be used as a delivery channel for delivering various auxiliary substances to tissue during the tissue operation. Fig.4 illustrates such an exemplary embodiment wherein the cartridge assembly 1 has been assembled to the surgical stapler 10 for stapling tissue.

As shown in Fig.4, the cartridge assembly 1 advances towards the anvil 5 under action of the driving member of the surgical stapler 10 to clamp tissue between the cartridge assembly 1 and the anvil 5, and then staples are fired into the clamped tissue upon the actuation of a firing member to perform stapling. To further improve the tissue operation effect, the cartridge assembly 1 may be fluidly communicated with a container 7 via one or more pipes 6. Auxiliary substances housed in the container 7 may flow into the cartridge assembly 1 through the pipes 6 as needed. Then the substances may be applied to the target tissue clamped between the cartridge assembly 1 and the anvil 5 through the staple-containing slots 24 formed on the forward surface 23 and openings 42 formed on the casing 4, in order to achieve various auxiliary functions. Although the container 7 is disposed outside the surgical stapler as shown in the figure, container 7 may be disposed inside the surgical stapler. In one embodiment, the container 7 may be arranged within a handle housing of the surgical stapler and provided with a driving mechanism such as a piston-cylinder driving mechanism, the driving mechanism operatively connected to the firing member of the surgical stapler, such that the actuation of the firing member also causes the actuation of the driving mechanism to force the substances housed in container 7 into cartridge assembly 1 through the pipes 6. Other arrangements for driving the substances in container 7 into cartridge assembly 1 are also possible. It can be appreciated that various valves can be provided on the pipes 6 for flow control.

The auxiliary substances in the container 7 are biocompatible and can be anti-microbial agents, hemostats, adjunct-therapy (medicaments facilitating recovery of a lesion of a patient or treatment of diseases), sealants or other medical materials. The hemostat, for example can be a combination of thrombin and fibrinogen. When the target tissue (e.g., a wounded site) is clamped between the cartridge assembly 1 and the anvil 5, the auxiliary substances such as hemostat can be applied to the target tissue through openings 42 provided as delivery channel on the casing 4 to promote tissue healing or assist in realization of other operation objects.

Although the present disclosure has been described with respect to the disposable cartridge assembly, the cartridge assembly can be designed to be used multiple times. For example, each components of cartridge assembly such as cartridge, staple driver and casing may be formed as an integral, one piece, which is for example molded of plastics. In either case, the assembly can be reconditioned for reuse after at least one use. Reconditioning can include any combination of the steps of disassembly of the assembly, followed by cleaning or replacement of particular pieces, and subsequent reassembly. In particular, the assembly can be disassembled, and any number of the particular pieces or parts of the assembly can be selectively replaced or removed in any combination. Upon cleaning and/or replacement of particular parts, the assembly can be reassembled for subsequent use either at a reconditioning facility, or by a surgical team immediately prior to a surgical procedure. Those skilled in the art will appreciate that reconditioning of an assembly can utilize a variety of techniques for disassembly, cleaning and/or replacement, and reassembly. Use of such techniques, and the resulting reconditioned instrument, are all within the scope of the present disclosure. Preferably, the assembly described herein will be processed before surgery. First, a new or used assembly is obtained and cleaned if necessary. The assembly can then be sterilized. In one sterilization technique, the assembly is placed in a closed and sealed container, such as a plastic or TYVEK® bag. The container and assembly are then placed in a field of radiation that can penetrate the container, such as gamma radiation, x-rays, or high-energy electrons. The radiation kills bacteria on the assembly and in the container. The sterilized assembly can then be stored in the sterile container. The sealed container keeps the assembly sterile until it is opened in the medical facility.

While this disclosure has been described as having exemplary designs, the present disclosure may be further modified within the scope of the disclosure. This application is therefore intended to cover any variations, uses, or adaptations of the present disclosure using its general principles.

## Claims

1. A cartridge assembly (1), comprising:
a cartridge (2) having a plurality of staple-containing slots (24), which extend longitudinally through a forward surface (23), for containing surgical staples;
a staple driver (3) with a plurality of tips (32) for driving the surgical staples; and
a casing (4) for housing the staple driver,
**characterized in that** the side of the casing is provided with one or more openings (42) for delivering a medium to the plurality of tips.

2. The cartridge assembly of claim 1, **characterized in that** the cartridge assembly is disposable.

3. The cartridge assembly of claim 1, **characterized in that** the cartridge assembly is reusable.

4. The cartridge assembly of claim 1, **characterized in that** the openings are provided on one or both sides of the casing.

5. The cartridge assembly of any one of claims 1-4, **characterized in that** the medium is a lubricant.

6. The cartridge assembly of any one of claims 1-4, **characterized in that** the openings are in fluid communication with a container for containing auxiliary substances via one or more pipes when the cartridge assembly is loaded into a surgical stapler.

7. The cartridge assembly of claim 6, **characterized in that** the auxiliary substances may include anti-microbial agents, hemostats, adjunct-therapy, sealants or a combination thereof.

## Patentansprüche

1. Magazinanordnung (1), umfassend:
ein Magazin (2) mit einer Vielzahl von Klammern enthaltenden Schlitzen (24), die sich in Längsrichtung durch eine vorwärtige Fläche (23) erstrecken und chirurgische Klammern enthalten,
einen Klammertreiber (3) mit einer Vielzahl von Spitzen (32) zum Antreiben der chirurgischen Klammern und
ein Gehäuse (4) zur Unterbringung des Klammertreibers,
**dadurch gekennzeichnet, dass**
die Seite des Gehäuses mit einer oder mehreren Öffnungen (42) zum Zuführen eines Mediums zu der Vielzahl von Spitzen versehen ist.

2. Magazinanordnung nach Anspruch 1, **dadurch gekennzeichnet, dass** die Magazinanordnung eine Einwegmagazinanordnung ist.

3. Magazinanordnung nach Anspruch 1, **dadurch gekennzeichnet, dass** die Magazinanordnung wiederverwendbar ist.

4. Magazinanordnung nach Anspruch 1, **dadurch gekennzeichnet, dass** die Öffnungen an einer oder beiden Seiten des Gehäuses vorgesehen sind.

5. Magazinanordnung nach einem der Ansprüche 1 - 4, **dadurch gekennzeichnet, dass** das Medium ein Schmiermittel ist.

6. Magazinanordnung nach einem der Ansprüche 1 - 4, **dadurch gekennzeichnet, dass** die Öffnungen über eine oder mehrere Röhren in Fluidverbindung mit einem Behälter zur Aufnahme von Hilfsstoffen stehen, wenn die Magazinanordnung in ein chirurgisches Klammernahtgerät geladen ist.

7. Magazinanordnung nach Anspruch 6, **dadurch gekennzeichnet, dass** die Hilfsstoffe antimikrobielle Mittel, Hämostatika, Zusatztherapie, Versiegelungsmittel oder eine Kombination davon einschließen können.

## Revendications

1. Ensemble cartouche (1), comprenant :
une cartouche (2) ayant une pluralité de fentes contenant des agrafes (24) qui s'étendent longitudinalement à travers une surface avant (23) afin de contenir des agrafes chirurgicales ;
un dispositif d'enfoncement d'agrafes (3) avec une pluralité de pointes (32) pour enfoncer les agrafes chirurgicales ; et
un boîtier (4) pour recevoir le dispositif d'enfoncement d'agrafes,
**caractérisé en ce que**
le côté du boîtier est pourvu d'une ou plusieurs ouvertures (42) pour distribuer un milieu vers la pluralité de pointes.

2. Ensemble cartouche selon la revendication 1, **caractérisé en ce que** l'ensemble cartouche est jetable.

3. Ensemble cartouche selon la revendication 1, **caractérisé en ce que** l'ensemble cartouche est réutilisable.

4. Ensemble cartouche selon la revendication 1, **caractérisé en ce que** les ouvertures sont prévues sur un côté ou les deux côtés du boîtier.

5. Ensemble cartouche selon l'une quelconque des revendications 1 à 4, **caractérisé en ce que** le milieu est un lubrifiant.

6. Ensemble cartouche selon l'une quelconque des revendications 1 à 4, **caractérisé en ce que** les ouvertures sont en communication fluidique avec un récipient destiné à contenir des substances auxiliaires par le biais d'un ou plusieurs tuyaux lorsque l'ensemble cartouche est chargé dans une agrafeuse chirurgicale.

7. Ensemble cartouche selon la revendication 6, **caractérisé en ce que** les substances auxiliaires peuvent comprendre des agents antimicrobiens, des agents hémostatiques, un adjuvant thérapeutique, des agents de scellement ou une combinaison de ceux-ci.
